# EUROPÄISCHE PATENTANMELDUNG

(11) **EP 3 578 123 A1**
(43) Veröffentlichungstag der Anmeldung: **11.12.2019**
(21) Anmeldenummer: 18176855.7
(22) Anmeldetag: 08.06.2018
(51) Int. Cl.: A61B 17/68

(54) **OSTEOSYNTHESE-MONTAGESET SOWIE VERWENDUNG EINES EXZENTERVERBINDERS**

(71) Anmelder: Universität Basel, 4003 Basel (CH)
(72) Erfinder: STÜBINGER, Stefan, 4102 Binningen, BL (CH)
(74) Vertreter: Kleine, Hubertus

(57) **Zusammenfassung**

Ein Osteosynthese-Montageset umfassend ein Knochenimplantat (1), insbesondere ein künstliches Knochenimplantat, und zumindest einen zumindest zweiteiligen Exzenterverbinder (2), sowie eine Verwendung eines Exzenterverbinders.

## Beschreibung

Die vorliegende Erfindung betrifft ein Osteosynthese-Montageset sowie eine Verwendung eines Exzenterverbinders.

Der Einsatz von Osteosynthesen z.B. zur Befestigung einer künstlichen Knochenplatte an einem Knochen erfolgt derzeit auf verschiedene Art und Weise. Einerseits wird die Knochenplatte randseitig über den Knochen gelegt oder es werden Verbindungsbrücken zwischen den Knochenimplantaten gelegt welche mit Schrauben festgezogen werden. In beiden Fällen ist ein Überstand durch die Brücken oder die Knochenplatten zu erkennen.

In einem weiteren Fall kann eine Knochenplatte durch seitlich eingeführte Schrauben, sogenannten Knochenschrauben, mit der Schädelkalotte verbunden werden. Hier muss handwerklich sehr genau gearbeitet werden. Zudem kann es zu Situationen kommen bei denen nicht genug Knochenmaterial zur Verankerung der Schraube vorhanden ist.

Unter Berücksichtigung der vorgenannten Vorbetrachtung sind entsprechende Osteosynthese-Montagesets daher sehr teuer.

Ausgehend vom Vorgenannten ist es daher die Aufgabe der vorliegenden Erfindung ein stabiles und einfach-zu montierendes Osteosynthese-Montageset bereitzustellen.

Die vorliegende Erfindung löse diese Aufgabe durch ein Osteosynthese-Montageset mit den Merkmalen des Anspruchs 1 und durch eine Verwendung mit den Merkmalen des Anspruchs 15.

Die vorliegende Erfindung betrifft ein Osteosynthese-Montageset. Es umfasst zumindest ein Knochenimplantat. Dabei kann es sich um ein künstliches Knochenimplantat z.B. aus Magnesiumoxid, PEEK (Polyetheretherketon), Metall (z.B. Edelstahl oder Titan) oder resorbierbaren Komponenten (z.B. Polylactid) handeln. Dieses künstliche Knochenimplantat kann vorzugsweise patientenspezifisch angefertigt werden kann. Allerdings kann ein Knochenimplantat in einer weiteren Ausführungsvariante auch ein körpereigener Knochen sein, welcher vor dem operativen Eingriff entsprechend bearbeitet wurde, z.B. durch Einfügen von Löchern zum Einsetzen von Exzentern.

Weiterhin umfasst das Osteosynthese-Montageset einen oder vorzugsweise mehrere Exzenterverbinder. Exzenterverbinder sind aus dem Bereich des Möbelbaus bekannt. Sie umfassen typischerweise einen Exzenter und einen Bolzen, insbesondere einen Schraubbolzen. Da das Prinzip des Exzenterverbinders auch auf andere mechanische Verbindungsmittel anwendbar sind, welche einen Kopf aufweisen, in welche der Exzenter eingreifen kann, ist als Exzenterverbinder im Rahmen der vorliegenden Erfindung auch eine Kombination aus Schraube mit einem Schraubenkopf und Exzenter zu verstehen.

Es ist aus dem Bereich des Möbelbaus zudem klar, dass ein Exzenterverbinder stets zumindest zweiteilig aufgebaut sein kann. Weiterhin kann vorteilhaft auch eine Abdeckkappe vorgesehen sein, wie sie analog auch im Möbelbau eingesetzt wird.

Aufgrund der Drehung des Exzenters bei der Montage unter Eingriff mit dem Schrauben- oder Bolzenkopf wird ein zusätzliches Anziehen des Exzenterverbinders erreicht und somit ein Zugschraubenprinzip erzeugt. Dies hat im Medizinbereich besondere Vorteile, denn dadurch wird das Knochenimplantat näher an den Knochen oder ein weiteres Knochenimplantat herangeführt und die Lücke oder Frakturstelle zwischen beiden Elementen zusätzlich verkleinert. Dies ergibt einen festen und sicheren Verbund zur Osseointegration und fördert somit die Heilung.

Darüber hinaus ist das Montageprinzip für den Operateur aufgrund der weitverbreiteten Anwendung des Exzenterverbinders in anderen Anwendungsbereichen insgesamt schon bekannt, so dass die Arbeitsschritte bei der Operation quasi selbsterklärend sind.

Vorteilhafte Ausgestaltungen der Erfindung sind Gegenstand der Unteransprüche.

Es ist von Vorteil, wenn der Exzenterverbinder zumindest zweiteilig ist umfassend einen Bolzen oder eine Schraube, zum Einsatz in einen Knochen, mit einem Bolzenkopf oder Schraubenkopf und einen Exzenter zum Einsatz in dem Knochenimplantat, wobei der Exzenter eine Innenspirale aufweist, zum Eingriff mit dem Bolzenkopf oder Schraubenkopf.

Das Knochenimplantat kann ein künstliches patientenindividuell gefertigtes Knochenimplantat, vorzugsweise aus einem Kunststoff oder eine Keramik, sein.

Bevorzugt ist das Knochenimplantat als Knochenplatte ausgebildet.

Der Bolzen kann vorzugsweise als Schraubbolzen mit einer Werkzeugaufnahme ausgebildet sein.

Der Bolzen oder die Schraube und/oder die Exzenter aus einem resorbierbaren Material oder aus Stahl oder Titan ausgebildet sein.

Es ist von Vorteil wenn der Bolzen oder die Schraube, der Exzenter, eine Abdeckkappe zum Abdecken der Werkzeugaufnahme und/oder eine Hülse entlang des Bolzens ein Release- oder Wirkstoffsystem zur Förderung der Einheilung und/oder Desinfektion aufweist. Die Abdeckkappe kann dabei einem unbeabsichtigten Lösen des Exzenters entgegenwirken.

Das Knochenimplantat weist insbesondere Löcher zur Aufnahme von Exzentern auf.

Die Exzenter können im Bereich der Innenspirale eine oder mehrere Rastnasen zur Verrastung der Bolzen während einer Schraubbewegung aufweisen.

Das Knochenimplantat kann vorzugsweise durch zumindest eine DreipunktAbstützung erreicht werden. Daher umfasst das Osteosynthese-Montageset zumindest drei Bolzen oder Schrauben und drei Exzenter.

Das Knochenimplantat umfasst vorteilhaft eine oder mehrere innenseitige Auflageflächen zur Vorzentrierung des Knochenimplantats bei dessen Auflage auf die Bolzen. Diese können mit geringem Übermaß den Konturen der Bolzen oder Schrauben entsprechend oder zumindest deren Schraubenköpfe oder Bolzenköpfe.

Das Osteosynthese-Montageset kann zudem eine Bohrschablone zur Positionierung von Sacklöchern in einem Knochen, insbesondere im Rand einer Öffnung in einer Schädelkalotte, aufweisen.

Alternativ oder zusätzlich kann eine Vergussmasse im Osteosynthese-Montageset enthalten sein, zum Vergießen der Exzenter mit dem Knochenimplantat. Es kann sich z.B. um einen Expositharz handeln. Auch resorbierbares Material und Material mit dem vorgenannten Release- und/oder Wirkstoffsystem kann in der Vergussmasse vorgesehen sein.

Alternativ oder zusätzlich kann auch ohne Vergussmasse gearbeitet werden, so dass die Exzenterverbinder lösbar sind und das Knochenimplantat bei Bedarf wieder entnommen werden darf.

Das Knochenimplantat kann vorteilhaft multimodal aufgebaut sein.

Ebenfalls erfindungsgemäß ist die Verwendung eines Exzenterverbinders zur Festlegung zweier Knochen, insbesondere zumindest eines künstlichen Knochenimplantats mit einem natürlichen Knochens. Auch Verbindungen zwischen einem natürlichen Knochen, also einem natürlichen Knochenimplantat und einem Knochen oder zwischen zwei Knochenimplantaten sind möglich.

Das Knochenimplantat kann multimodal also mehrteilig aufgebaut sein.

Vorteilhaft können einzelne Teile oder sämtliche Teile des Osteosynthese-Montagesets als Single-Use-Bauteile ausgebildet sein.

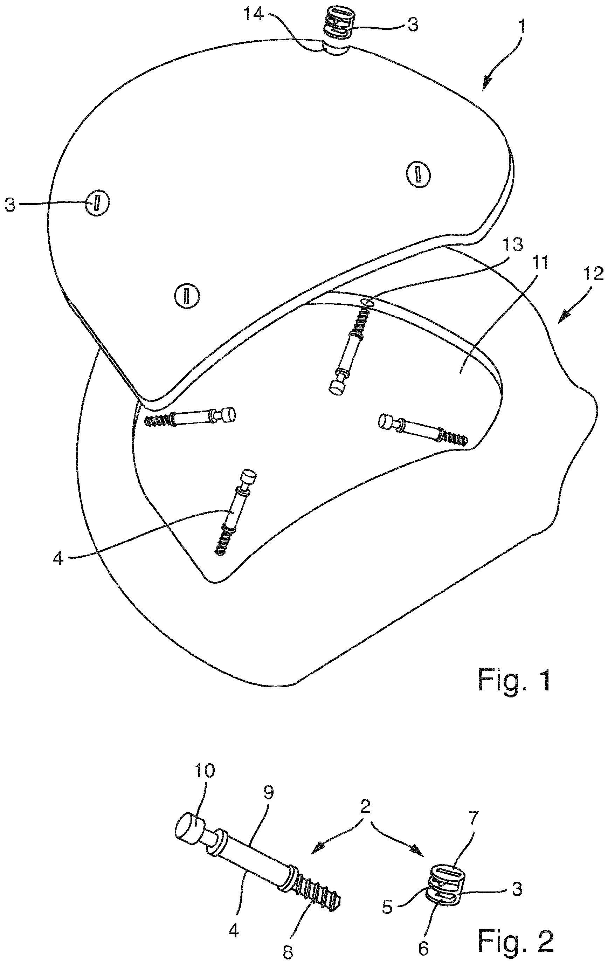

Nachfolgend wird die Erfindung anhand eines Ausführungsbeispiels unter Bezugnahme auf die nachfolgende Fig. 1 und 2 näher erläutert.

Es zeigen:
Fig. 1: ein erfindungsgemäßes Montagesystem umfassend eine vorgefertigte Schädelplatte und mehrere Exzenterverbinder; und
Fig. 2: einen bekannten Exzenterverbinder.

Fig. 1 zeigt eine Osteosynthese mit einem künstlichen Knochenimplantat 1 in Form einer Schädelplatte 1a. Diese kann vorzugsweise patientenindividuell hergestellt sein. Dieses kann analog zur der WO 2017 153 560 A1 bevorzugt wie folgt erfolgen:
A) Erfassen von Daten eines Patienten, für welchen die Schädelplatte als plattenförmiges Knochenimplantat bestimmt ist;
   Das Knochenimplantat kann aus einer einzelnen Schädelplatte oder zwei oder mehr Knochen- oder Knochenfragmente bestehen. Die Daten können vorzugsweise Aufnahmen eines bildgebenden Verfahrens sein. Üblicherweise werden diese Aufnahmen von bildgebenden Verfahren zur Diagnose genutzt. Im vorliegenden Fall werden diese Aufnahmen jedoch zur Erstellung eines Modells unabhängig von einer Diagnose erstellt oder alternativ nach erfolgter Diagnose weiterverwandt. Weitere Details zu bevorzugten Bildgebenden Verfahren können der WO 2017 153 560 A1 entnommen werden.
B) Erstellen eines Modells anhand der erfassten Daten;
   Das Erstellen des Modells kann als Computermodell erfolgen.
C) Erstellen von Fertigungsvorgaben für zumindest ein Knochenimplantat auf Grundlage des erstellten Modells wobei die Fertigungsvorgaben umfassen: C1) eine Dimensionierung des Knochenimplantats.
   Die Fertigungsvorgaben werden vorzugsweise anhand des Computermodells derart erstellt, dass die Teilelemente je nach ihrer Funktion unterschiedlich dimensioniert werden. Auch eine Knochenplatte ist an einer Stelle etwas dicker als an einer anderen. Die benötigte Materialdicke und Flexibilität der Knochenimplantatabschnitte können durch das Modell, welches auf patientenspezifischen Daten beruht, individuell ausgestaltet und gefertigt werden.
D) Fertigung des Knochenimplantats auf Basis der Fertigungsvorgaben; wobei auch die Teilelemente miteinander des Knochenimplantats montierbar sind.

Der Schritt der Fertigung von Teilelementen kann auch das Anpassen von vorgefertigten Elementen umfassen, beispielsweise ein Abschleifen einer Vorform anhand einer patientenspezifischen Fertigungsvorgabe.

Das Erstellen von Fertigungsvorgaben für ein Knochenimplantat erfolgt auf Grundlage des erstellten Modells, wobei die Fertigungsvorgaben folgende weitere Angaben umfassen:
C2) eine Materialauswahl bezüglich eines oder mehrerer Materialen für das Knochenimplantat,
   Dies ermöglicht eine optimale Abstimmung bezüglich einer erwünschten Flexibilität oder Starrheit der Teilelemente.
C3) eine Auswahl der Anzahl und/oder der Position von verwendeten Exzenterverbindern zur Verankerung im Körper.

Fig. 1 zeigt zusätzlich zur Schädelplatte einen Exzenterverbinder 2 umfassend einen Exzenter 3 und einen Schraubbolzen 4. Der Exzenter 3 ist zylinderförmig ausgebildet und weist einen Innenspirale 5 und eine Ausnehmung 6 zum Einführen eines Bolzenkopfes 10 auf. Weiterhin weist der Exzenter eine Werkzeugaufnahme 7, z.B. eine Schlitz-, eine Kreuzschlitz- und/oder eine Sechskantaufnahme oder dergleichen auf, in welche ein Werkzeug eingreifen kann.

Durch Verdrehen des Exzenters 3, z.B. um 90°, 180° oder mehr, wird der Bolzenkopf 10 in Eingriff mit der Innenspirale 5 gebracht.

Die Innenspirale 5 ist in Fig. 1 und 2 als Doppelspirale ausgebildet, es kann sich allerdings auch um eine Einfachspirale handeln.

Der Schraubbolzen 4 weist in Fig. 4 ein Schraubsegment 8, einen Bolzenschaft 9 und einen endständigen Bolzenkopf 10 auf.

Die Schädelplatte 1a weist Löcher 14 zur Aufnahme der Exzenter 3 auf, derart, dass die im Exzenter 3 angeordnete Werkzeugaufnahme von außen, also außenseitig zur Schädelkalotte 12, zugänglich ist.

Beim Zusammensetzen der Schädelkalotte werden in den Rand der Öffnung 11 der Schädelkalotte 12 zunächst Sacklöcher 13 gebohrt. Der Rand der Öffnung 11 kann zudem vorbehandelt sein, um Hinterschneidungen, scharfe Ecken und dergleichen zu vermeiden.

Sodann werden die Schraubbolzen in die Sacklöcher 13 eingesetzt, derart, dass die Bolzenschäfte 9 und die Bolzenköpfe 10 radial nach innen in die Öffnung 11 der Schädelkalotte 12 hineinragen.

Die Schädelplatte wird sodann auf die Bolzenschäfte 9 und Bolzenköpfe 10 aufgesetzt. Während im Anwendungsbereich der Möbel oftmals die Möbelwände zusammengesteckt werden, ist hier lediglich ein Aufsetzen bzw. Auflegen der Schädelplatte beabsichtigt, da nicht mehr Platz vorhanden ist. Allerdings ist ein einseitiges Aufstecken z.B. auf zwei Schraubbolzen auch denkbar, so dass das Auflegen nur auf den zwei anderen Schraubbolzen erfolgt.

Weiterhin werden die Exzenter 3 in die Löcher 14 eingesetzt und verdreht, so dass die Innenspirale 5 in Eingriff mit dem jeweiligen Bolzenkopf kommt.

Abschließend kann eine nicht-dargestellte Abdeckkappe auf die Oberseite des Exzenters aufgesteckt werden, welcher Ablagerungen in der Werkzeugaufnahme verhindert.

In einer bevorzugten Ausführungsvariante kann die Innenspirale 5 einen oder mehrere Rastnasen aufweisen, als Verdrehsicherung der Exzenter 3.

Das Material der Schraubbolzen 4 und/oder der Exzenter 3 kann aus einem Metall, vorzugsweise Stahl, insbesondere Edelstahl, oder Titan bestehen.

Alternativ kann der Schraubbolzen 4 und/oder der Exzenter 3 auch aus Kunststoff wie z.B. PEEK bestehen und insbesondere aus einem resorbierbaren Kunststoff, z.B. PLA bestehen. Schraubbolzen 4 und/oder der Exzenter 3 oder weitere Bestandteile wie eine Abdeckkappe oder eine Vergussmasse können auch aus Magnesium bzw. Magnesiumoxid oder aus Hydroxylapatit bestehen.

Der Bolzenschaft 9 kann eine außen anliegende Hülse aufweisen. Diese Hülse oder die Abdeckkappe können aus einem resorbierbaren Kunststoff gefertigt sein, welcher mit einem Release- und/oder Wirkstoffsystem ausgerüstet, insbesondere beschichtet oder eingebunden, ist. Unter dem Begriff Wirkstoffe bzw. Wirkstoffsysteme sind Arzneistoffe zu verstehen. Dies können z.B. Antibiotika, Immunsuppressiva, Wachstumshormone oder Zytostatika sein. Releasesysteme umfassen alle Substanzen, welche typischerweise zusätzlich dem Arzneistoff zugegeben werden, beispielsweise um die Freisetzung des Arzneistoffes im Körper hinsichtlich des Abgabezeitraumes und des Abgabeortes zu steuern oder um den Arzneistoff unter verschiedenen Bedingungen haltbar zu machen. Typische Substanzen sind z.B. Tocopherol, welches oft als Antioxidans zugesetzt wird, oder Polysaccharide, z.B. Chitin, zur Mikroverkapselung eines entsprechenden Arzneistoffes. Die Wahl des Releasesystems hängt u. a. auch von der Wahl des Materials, also des Basismaterials, ab aus welchem die Hülse oder Abdeckkappe hergestellt werden soll. Auch der Exzenter 3 und/oder der Schraubbolzen 4 können ein solches Wirkstoff- und/oder Releasesystem aufweisen.

Die Sacklöcher 13 können vorzugsweise leicht schräg gebohrt werden, um eine bessere Zugänglichkeit der Schraubbolzen zu erlauben.

Fig. 2 zeigt nochmals die Exzenterverbinder 2 im Detail.

Zur besseren Festlegung der Schrauben kann ein Schlauch, z.B. ein Stahlschlauch mit einem Bit auf ein Werkzeug aufgesteckt werden.

Im Rahmen der vorliegenden Erfindung müssen nicht zwingend Schraubbolzen für den Exzenterverbinder genutzt werden. Es sind beispielsweise auch Steckbolzen oder aber eine Schraube denkbar.

Auf der abgewandten Seite der Knochenplatte, also auf der Innenseite, kann eine vorgefertigte Auflagefläche in der Kontur des Schraubbolzens angeordnet sein, welcher eine Vorzentrierung der Knochenplatte bei deren Auflegen auf die Schraubbolzen erlaubt.

Zur Positionierung der Sacklöcher 13 kann in einem Montageset neben der Schädelplatte und den mehreren zweiteiligen Exzenterverbindern zudem eine Bohrhilfe, wie z.B. eine Bohrschablone, vorhanden sein.

Auch ein Werkzeug zum Einsetzen der Bolzen 4 und/oder ein Bohrer für die Sacklöcher 13 können im Montageset mitgeliefert werden.

### Bezugszeichen

- 1: Knochenimplantat
- 2: Exzenterverbinder
- 3: Exzenter
- 4: Schraubbolzen
- 5: Innenspirale
- 6: Ausnehmung
- 7: Werkzeugaufnahme
- 8: Schraubsegment
- 9: Bolzenschaft
- 10: Bolzenkopf
- 11: Öffnung
- 12: Schädelkalotte
- 13: Sackloch
- 14: Löcher

## Patentansprüche

1. Osteosynthese-Montageset umfassend ein Knochenimplantat (1), insbesondere ein künstliches Knochenimplantat, und zumindest einen zumindest zweiteiligen Exzenterverbinder (2).

2. Osteosynthese-Montageset nach Anspruch 1, **dadurch gekennzeichnet, dass** der Exzenterverbinder (2) zumindest zweiteilig ist, umfassend einen Bolzen (4) oder eine Schraube, zum Einsatz in einen Knochen, mit einem Bolzen- oder Schraubenkopf (10) und einen Exzenter (3) zum Einsatz in dem Knochenimplantat (1), wobei der Exzenter (3) eine Innenspirale (5) aufweist, zum Eingriff mit dem Bolzenkopf (10).

3. Osteosynthese-Montageset nach Anspruch 1 oder 2, **dadurch gekennzeichnet, dass** das Knochenimplantat (1) ein künstliches patienten-individuell gefertigtes Knochenimplantat, vorzugsweise aus einem Kunststoff, Metall oder einer Keramik, ist.

4. Osteosynthese-Montageset nach einem der vorhergehenden Ansprüche, **dadurch gekennzeichnet, dass** das Knochenimplantat (1) als Knochenplatte ausgebildet ist.

5. Osteosynthese-Montageset nach einem der vorhergehenden Ansprüche, **dadurch gekennzeichnet, dass** der Bolzen (4) als Schraubbolzen mit einer Werkzeugaufnahme (7) ausgebildet ist.

6. Osteosynthese-Montageset nach einem der vorhergehenden Ansprüche, **dadurch gekennzeichnet, dass** der Bolzen (4) und/oder die Exzenter (3) aus einem resorbierbaren Material oder aus Stahl oder Titan ausgebildet ist.

7. Osteosynthese-Montageset nach einem der vorhergehenden Ansprüche, **dadurch gekennzeichnet, dass** der Bolzen (4), der Exzenter (3), eine Abdeckkappe zum Abdecken der Werkzeugaufnahme (7) und/oder eine Hülse entlang des Bolzens (4) ein Release- oder Wirkstoffsystem zur Förderung der Einheilung und/oder der Desinfektion aufweist.

8. Osteosynthese-Montageset nach einem der vorhergehenden Ansprüche, **dadurch gekennzeichnet, dass** das Knochenimplantat (1) Löcher zur Aufnahme von Exzentern aufweist.

9. Osteosynthese-Montageset nach einem der vorhergehenden Ansprüche, **dadurch gekennzeichnet, dass** der oder die Exzenter (3) im Bereich der Innenspirale (5) eine oder mehrere Rastnasen zur Verrastung der Bolzen während einer Schraubbewegung aufweist.

10. Osteosynthese-Montageset nach einem der vorhergehenden Ansprüche, **dadurch gekennzeichnet dass** das Osteosynthese-Montageset zumindest drei Bolzen (4) oder Schrauben und drei Exzenter (3) aufweist.

11. Osteosynthese-Montageset nach einem der vorhergehenden Ansprüche, **dadurch gekennzeichnet, dass** das Knochenimplantat (1) innenseitige Auflageflächen zur Vorzentrierung des Knochenimplantats (1) bei dessen Auflage auf die Bolzen (4) oder Schrauben aufweist.

12. Osteosynthese-Montageset nach einem der vorhergehenden Ansprüche, **dadurch gekennzeichnet, dass** das Osteosynthese-Montageset eine Bohrhilfe, insbesondere eine Bohrschablone, zur Positionierung von Sacklöchern (13) in einem Knochen, insbesondere im Rand einer Öffnung (11) in einer Schädelkalotte (12), und/oder ein Werkzeug zum Einsetzen der Bolzen (4) oder Schrauben und/oder einen Bohrer aufweist.

13. Osteosynthese-Montageset nach einem der vorhergehenden Ansprüche, **dadurch gekennzeichnet, dass** der Exzenterverbinder lösbar ausgebildet ist.

14. Osteosynthese-Montageset nach einem der vorhergehenden Ansprüche, **dadurch gekennzeichnet, dass** das Osteosynthese-Montageset zudem eine Vergussmasse umfasst, mit welchem der Exzenter nach der Montage vergossen werden kann.

15. Verwendung eines Exzenterverbinders (2) nach einem der vorhergehenden Ansprüche zur Festlegung zweier Knochen, insbesondere zumindest eines künstlichen Knochens mit einem natürlichen Knochen.
